(19) Europäisches Patentamt
European Patent Office
Office européen des brevets

(11) Publication number: **0 078 818**
**B1**

(12) # EUROPEAN PATENT SPECIFICATION

(45) Date of publication of patent specification: **21.11.84**

(51) Int. Cl.[3]: **C 07 C 27/00,** C 07 C 43/04, C 07 C 53/02

(21) Application number: **82901376.2**

(22) Date of filing: **07.05.82**

(86) International application number: **PCT/NO82/00024**

(87) International publication number: **WO 82/04041 25.11.82 Gazette 82/28**

(54) **PROCESS FOR CO-PRODUCTION OF TERT. ALKYL-METHYLETHER AND FORMIC ACID.**

(30) Priority: **13.05.81 NO 811629**

(43) Date of publication of application: **18.05.83 Bulletin 83/20**

(45) Publication of the grant of the patent: **21.11.84 Bulletin 84/47**

(84) Designated Contracting States: **AT BE CH DE FR GB LI LU NL SE**

(56) References cited: **none**

(73) Proprietor: **ONSAGER, Olav-T**
**Arkt. Christies gt. 8**
**N-7000 Trondheim (NO)**

(72) Inventor: **ONSAGER, Olav-T**
**Arkt. Christies gt. 8**
**N-7000 Trondheim (NO)**

(74) Representative: **Skailes, Humphrey John et al**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 078 818 B1

## Description

This invention relates to a process for the co-production of tert.alkyl-methyl-ether and formic acid.

It is previously known to prepare methyl-tert.butyl-ether (MTBE) and tert.amyl-methyl-ether (TAME) by addition of methanol to the corresponding olefines, isobutene and isoamylene (Hydrocarbon Processing, March 1981, 89—94). MTBE and TAME are both known as advantageous gasoline additives to improve the octane number. Thus, it is reasonable to assume that the market for these products will develop in a favourable direction with respect to the future industrial production.

Further, it is known to prepare formic acid from methyl formate either by direct ester hydrolysis or indirect hydrolysis via formamide as intermediate. The direct hydrolysis is characterized in unfavourable equilibrium conditions in the hydrolysis reaction, which entail great expenses in connection with the separation of the products and recovery of pure formic acid. The industrial hydrolysis via formamide as intermediate with subsequent liberation of formic acid by addition of mineral acid ($H_2SO_4$), is connected with a heavy economical burden when stoichiometric amounts of ammonia and mineral acid are used in the process. By-production of ammonium salt is today also highly undesirable in view of environment and energy considerations.

The process according to the invention for the co-production of tert.alkyl-methyl-ether and formic acid is characterized in that the tertiary alcohols tert.butanol and/or tert.amyl-alcohol are reacted with methyl formate in an acid-catalysed chemical reaction. The chemical reaction is defined by equation 1, in which $R = CH_3$ or $C_2H_5$.

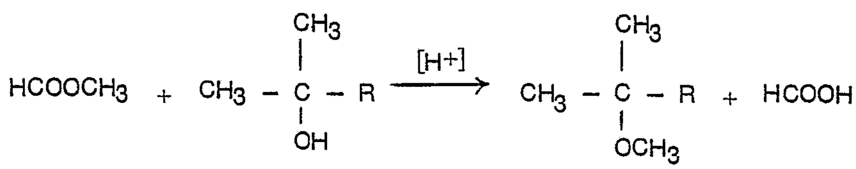


Instead of or together with the stated tert.alcohols it is also possible to use as starting materials those olefines which are obtained by removing one molecule of water, viz. isobutene, 2-methyl-butene-2 and 2-methyl-butene-1, in the presence of water.

In the process according to the invention, there are co-produced two types of valuable products which may easily be separated from each other by fractional distillation without the formation of undesired by-products. This fact makes the process technically and economically advantageous in comparison with the known methods for the preparation of the two types of products separately.

The reaction according to the invention as illustrated in equation 1, takes place in the liquid phase in the presence of acid catalysts. Surprisingly, it has been found that the desired reaction takes place with high yields and high selectivity. Only minor amounts of the expected re-esterification products, tert.butyl formate and tert.amyl formate, are formed in the system. Further, these compounds may readily be separated from the desired products by fractional distillation and may be recycled to the reaction for further conversion. As catalysts Bronsted acids of different types are used. Both mineral acids such as HF, $H_2SO_4$ and $H_3PO_4$, organic acids such as p-toluene sulfonic acid, methane sulfonic acid and trifluoro methane sulfonic acid, $H^+$-ion exchangers such as Amberlyst 15, solid inorganic acids such as e.g. $Al_2O_3$, $SiO_2$, $Al_2O_3{\cdot}SiO_2$ and $TiO_2$ and super-acids (combination of Bronsted and Lewis acids) such as $HF{\cdot}BF_3$, $HF.TaF_5$, $TiO_2{\cdot}H_2SO_4$ and $HCl{\cdot}AlCl_3$ may be used. It is particularly advantageous to use catalysts which are insoluble in the reaction mixture.

The reaction takes place at a temperature in the range of 50°C to 200°C. Most advantageously, a temperature of about 100°C is used. The pressure in the reaction zone is chosen so that a substantial proportion of the reactants are present in the liquid phase. Preferably pressures below 50 atmospheres are chosen. The ratio between the reactants may be varied within wide limits. It is preferably chosen to obtain maximum conversion to the desired products. The reaction takes place without the use of solvent, although inert organic solvents may of course be used. During the conversion in the reaction zone the tertiary alcohols are to some extent decomposed into water and the corresponding mono-olefines, while at the same time the tertiary alcohols will be formed from these compounds if the thermodynamic conditions favour this. A further advantage of the process according to the invention is accordingly that the iso-olefines, isobutene, 2-methyl-butene-2 and 2-methylbutene-1 together with water may be fed to the reaction zone as starting material which is equivalent to the tertiary alcohols, tert.butanol and tert.amyl alcohol.

In addition to the pure iso-olefines, it is also possible to use mixtures of iso-olefines with n-olefines and saturated hydrocarbons, since only the iso-products are selectively converted in the process according to the invention. Under specific circumstances this may represent a clear technical and economical advantage, since

the mentioned iso-olefines are readily available products from large hydrocarbon cracking plants in admixture with such products.

The prepared products, MTBE and TAME represent most valuable gasoline additives having a "Reasearch Octane Number" (RON) of 118 and 112 respectively, and may be added directly to current gasoline products. Formic acid, which is the strongest organic carboxylic acid, is employed to a great extent as neutralizing agent in the industry and as silo liquid.

The following examples will illustrate the invention further.

Example 1

4.0 grams of methyl formate and 5.0 grams of tertiary butanol were reacted in a microreactor (20 ml) made of acid resistant steel (SS 316) with magnetic stirrer at 90°C/30 bar nitrogen pressure in the presence of 0.3 grams of acidic ion exchanger of the type Amerlyst 15. After a reaction time of 16 hours, the reaction mixture was cooled to 0° and analysed by means of gas/liquid-chromatography (g.l.c.). In addition to unreacted starting compounds and minor amounts of tert.butyl formate, isobutene, water and methanol, the reaction mixture contained 11% of formic acid and 18% MTBE

Example 2

4.0 grams of methyl formate and 6.0 grams tert.amylalcohol were reacted in the same reactor as described in Example 1 and 80°C/30 bar nitrogen pressure in the presence of 1.0 gram of para-toluene sulfonic acid catalyst. After a reaction time of 2 hours the mixture was cooled to 0°C, the catalyst was neutralized and the mixture was analysed by g.l.c. In addition to unreacted starting compounds and minor amounts of tert.amyl formate, isoamylene, water and methanol, the reaction mixture contained 8% of formic acid and 10% of TAME.

Example 3

The reaction described in Example 1 was repeated with the exception that instead of 5.0 grams tert.butanol (about 67.5 mM) a corresponding amount of isobutene (67.5 mM) and water (67.5 mM) were used. This experiment also yielded about 11% of formic acid and 18% of MTBE, illustrating that the tertiary alcohol and a corresponding iso-olefin/$H_2O$ are equivalent starting materials.

Example 4

0.2 kilogram of methyl formate and 0.25 kilogram of tert.butanol were reacted in a 1-liter autoclave made of acid resistant steel (SS 316) having a mechanical stirrer and electric temperature control at 100°C/30 bar ($N_2$) in the presence of 20 grams of Amberlyst 15 as catalyst. After a reaction time of 5 hours the reaction mixture was cooled to 0°C, and the heterogeneous catalyst was separated from the liquid phase. The liquid reaction product was subjected to fractional distillation at approximately atmospheric pressure. 60 grams of MTBE were recovered as a fraction in the temperature range of 53°C to 58°C, and 45 grams of formic acid (mixed with 17 grams of water) were recovered as a fraction having a boiling point above 90°C. Unreacted methyl formate together with isobutene, methanol and minor amounts of MTBE were recovered as a low-boiling fraction at a temperature up to 53°C. The fraction between 58°C and 90°C consisted primarily of unreacted tert.butanol together with minor amounts of tert.butyl formate. With the exception of the MTBE and formic acid fractions, all the products were used as starting materials in a new experiment under the same reaction conditions together with fresh methyl formate, tert.butanol and water in an amount corresponding to the amounts of products which were isolated, so that the molar composition in the new experiment was identical to that of the first reaction. The same catalysts were also used. The results of the repeated experiment illustrated that both MTBE and formic acid may be produced at a selectivity of about 90% by recycling unreacted methyl formate and tert.butanol together with the products, isobutene, methanol and tert.butyl formate to the reaction zone.

## Claims

1. Process for co-production of tert-$C_{4-5}$-alkyl-methyl-ether and formic acid, characterized in that a tert-$C_{4-5}$-alkanol and/or an olefin corresponding to said alkanol and water is reacted with methyl formate in a reaction zone in the presence of an acidic catalyst, whereafter the desired products are recovered from the obtained reaction mixture, and the rest of the reaction mixture is optionally recycled to the reaction zone together with fresh starting materials.

2. Process according to claim 1, characterized in that a catalyst which is insoluble in the reaction mixture, is used as acidic catalyst.

## Revendications

1. Procédé de production simultanée d'éther méthylique de tert. alkyle en $C_{4-5}$ et d'acide formique, caractérisé en ce que l'on fait réagir un tert-alcanol en $C_{4-5}$ et/ou une oléfine correspondant à cet alcanol et de l'eau sur du formiate de méthyle dans une zone de réaction en présence d'un catalyseur de caractère acide, après quoi on récupère les produits désirés du mélange réactionnel obtenu, et le cas échéant on recycle le reste du mélange réactionnel dans la zone de réaction ensemble avec de nouveaux produits de départ.

2. Procédé selon la revendication 1, caractérisé en ce que l'on utilise un catalyseur insoluble dans le mélange réactionnel en tant que catalyseur de caractère acide.

## Patentansprüche

1. Verfahren zur gemeinsamen Herstellung von tert.—$C_{4-5}$-Alkylmethyläther und Ameisensäure, dadurch gekennzeichnet, daß ein tert.—$C_{4-5}$-Alkanol und/oder ein Olefin entsprechend diesem Alkanol und Wasser mit Ameisensäuremethylester in einer Reaktionszone in Gegenwart eines sauren Katalysators umgesetzt werden, wonach die gewünschten Produkte aus der erhaltenen Reaktionsmischung gewonnen werden und der Rest der Reaktionsmischung-gegebenenfalls zu der Reaktionszone zusammen mit frischen Ausgangsmaterialien zurückgeführt wird.

2. Verfahren gemäß Anspruch 1, dadurch gekennzeichnet, daß ein Katalysator, der in dem Reaktionsgemisch unlöslich ist, als saurer Katalysator verwendet wird.